# EUROPÄISCHE PATENTSCHRIFT

(11) **EP 1 789 563 B1**
(45) Veröffentlichungstag und Bekanntmachung des Hinweises auf die Patenterteilung: **14.12.2011**
(21) Anmeldenummer: 05786884.6
(22) Anmeldetag: 02.09.2005
(51) Int. Cl.: C12N 15/82

(54) **WURZEL- UND XYLEMPARENCHYMSPEZIFISCHER PROMOTOR**
ROOT-SPECIFIC AND XYLEM PARENCHYMA-SPECIFIC PROMOTER
PROMOTEUR SPECIFIQUE AUX RACINES ET SPECIFIQUE AU PARENCHYME DU XYLEME

(30) Priorität: 03.09.2004 DE 102004043207
(43) Veröffentlichungstag der Anmeldung: 30.05.2007
(73) Patentinhaber: KWS Saat AG, 37555 Einbeck (DE); Südzucker AG Mannheim/Ochsenfurt, 68165 Mannheim (DE)
(72) Erfinder: HEHL, Reinhard, 38114 Braunscheig (DE); OLTMANNS, Heiko, 26835 Neukamperfehn (DE); STAHL, Dietmar Jürgen, 37574 Einbeck (DE)
(74) Vertreter: Pohl, Manfred
(86) Internationale Anmeldenummer: PCT/DE2005/001540
(87) Internationale Veröffentlichungsnummer: WO 2006/024291

(56) Entgegenhaltungen:
- EP-A- 1 207 204
- DE-C5-102004 043 207
- SAKUTA CHIYOKO ET AL: "Vascular tissue-specific gene expression of xylem sap glycine-rich proteins in root and their localization in the walls of metaxylem vessels in cucumber" PLANT AND CELL PHYSIOLOGY, Bd. 41, Nr. 5, Mai 2000 (2000-05), Seiten 627-638, XP009067735 ISSN: 0032-0781
- KLOOS DOROTHEE U ET AL: "Isolation and molecular analysis of six taproot expressed genes from sugar beet" JOURNAL OF EXPERIMENTAL BOTANY, Bd. 53, Nr. 373, Juni 2002 (2002-06), Seiten 1533-1534, XP002385131 ISSN: 0022-0957
- CHEADLE, C. ET AL: "Control of gene expression during T cell activation: alternate regulation of mRNA transcription and mRNA stability", BMC GENOMICS, vol. 6, no. 75, 2005,
- SIEBURTH, L.E. AND MEYEROWITZ, E.M.: "Molecular dissection of the AGAMOUS control region shows that cis elements for spatial regulation are located intragenically", THE PLANT CELL, vol. 9, March 1997 (1997-03), pages 355-365,
- ELLERSTROEM M ET AL: "FUNCTIONAL DISSECTION OF A NAPIN GENE PROMOTER: IDENTIFICATION OF PROMOTER ELEMENTS REQUIRED FOR EMBRYO AND ENDOSPERM-SPECIFIC TRANSCRIPTION", PLANT MOLECULAR BIOLOGY, SPRINGER, DORDRECHT, NL, vol. 32, no. 6, 1 January 1996 (1996-01-01), pages 1019-1027, XP001007602, ISSN: 0167-4412, DOI: DOI:10.1007/BF00041385

### SEQUENZPROTOKOLL

<110> KWS SAAT AG
<120> wurzel- und xylemparenchymspezifischer Promotor
<130> KWS0087
<140>
   <141>
<160> 2
<170> PatentIn Ver. 2.1
<210> 1
   <211> 2502
   <212> DNA
   <213> Beta vulgaris
<220>
   <221> TATA_signal
   <222> (2462) .. (2467)
<400> 1
   aagcttctcc tttgagaagg agaagtcagt attacccgtt caaaagggta ttgactattg 60
   agtcaccgag gaatgcgact cttccaagaa tcatgattca agaattccat gtgacaaaag 120
   gtcacgggaa ctagttaaag tgcaagttcc aaatctcgct tgtttttcgt ccctccgatt 180
   gttaaatcag tccactcctt cctcatcttc catcattgtt atagtgtttg tcaaccatca 240
   acaaaagaac ttcaatacac ttatttcaca atacggacct ctagattgag cagctttatc 300
   taagtttgag ttatttatgt tattgtaatt gttatgtaac aacaagtaaa gctcaagtta 360
   gtttctttgt ttttatttgg ttgcattagc gttaggatgt actttcattt ttaattaatg 420
   caaaattctc cgttataaaa aaaaagggat ccctctatgc cttcataaac tgcctaattt 480
   ctgcaattga gatcccaaaa tagaaacaat gaatttgaaa cctaccaatt tgagtgctgt 540
   gcctaagtcc taagacccaa gtcatctcta atgacacacc caatctcacc gagtagatag 600
   gtgatcacta gaaaatagag ataaatatga aacaattttc ttctttttgt gtaatttcaa 660
   aagaaatatt tcacttgttt tagaacggat atatatatat atatatatat atatatatat 720
   atatatatat atatatatat ataaaagctc attagaaatt tgataataga actagtactt 780
   ttttaatgta tcttgatggg ataaccttag cttgcaaaag cacactgacg ttggaatgct 840
   gtcgtttgat gacattggca acatttcctt tcaatttttt cttccattat ctcattaatc 900
   cactaggata ctagcctaat ggcccattaa tggtcttcat tggaaaaatg tattataaaa 960
   tttattcttc atggaaatat aagaaaaggc cttctaatac tcccactctc ccagtgttgg 1020
   aaagaaagtt gggcagataa agtttggagc tccaatcctt caagtcattt gtgactgaat 1080
   actgtttatg ttatgttaat gggttaggct tgatgatcca aaaggtaaca aaggaaagtt 1140
   ctacaatatc tcgcgaagat ttagttatag agcttagtat aaaactgtat catattgaga 1200
   atctattttt catggggata ccgtatggag tcttagtggt catttggttg catacggaaa 1260
   attacacttc ttagcaatat gtaattccca tgaaatttca tttcttatga agagtacata 1320
   actgttttgg ttatatactt ttactatttt atgagaagtg aaattccaga aatttgtgtg 1380
   tttttgttaa ccaaacaatc cttcatcttt atatttaaca agaacttaca tttcctatgt 1440
   tttgtagtat caactaaacg attacttatt cttgagtcaa tgtttaaatt taatactagg 1500
   tagtgtgtat ttatattatt atatacgagt aattttctta tgatggtgca aataataatc 1560
   tttttaataa aattgatgag tacatattga actatacgtg ttgatgtgcg gatacttcta 1620
   aataagtttt atatacaact ttcttatgca tcacttcatt taataagaac aacatatgat 1680
   agcctagtgg tacattgaca taacatctga tattagaggt cccgggatcg agtcttacct 1740
   tttgtgagaa ggatcctcaa ttttttccct tcttgtaact tgcaagagac aaatcacttc 1800
   ttttgatgat agatgatgcg atattatgag aaatgtcata cacaagataa ataattccat 1860
   ttttactcga taatatataa ctcataattg tttctgataa ttcttatcaa taatatactc 1920
   cctccgtcct ctattagttt acccgtattc cttttaagag tgtctcttat tagtttaccc 1980
   ctttttattt tttgctattt ttggaatggg accacaaact tttcatcaca cataaattca 2040
   atttaatata aaataatagt ttctctttct ttatatgacc cacaatatta tttctcctaa 2100
   aacttgtgcg aaagaaaggg gtaaactaat agaggacgga gggagtacat cattagtaat 2160
   atatgggtac tcatggtatg catagcgcat tttccatgca ataatattgc aatattgatc 2220
   gactgatcaa catgcaaatc aaatcaccaa tcacaactcg gaactaaggt agtatttgac 2280
   cccaatttat actggggaag tttcactttc agaaatacgt aaacacaaaa acaagtgcgt 2340
   gcatgcgcat tggccattgg agtattgaag tatatcttgt agaggataag caatgtcttg 2400
   gtctttccaa tatcaatata tcattactta gtaatgatat ggtctgcctt ttcctatgct 2460
   atataaaccc tcaaatcctc taactctaaa acccaccaaa tc 2502
<210> 2
   <211> 1151
   <212> DNA
   <213> Zea mays
<220>
   <221> TATA signal
   <222> (16) .. (24)
<220>
   <221> intron
   <222> (129) .. (1133)
<220>
   <221> promoter
   <222> (1) .. (1147)
<400> 2
   cttcctcgcc cgccgtaata aatagacacc ccctccacac cctctttccc caacctcgtg 60
   ttgttcggag cgcacacaca cacaaccaga tctcccccaa atccacccgt cggcacctcc 120
   gcttcaaggt acgccgctcg tcctcccccc ccctctctac cttctctaga tcggcgttcc 180
   ggtccatggt tagggcccgg tagttctact tctgttcatg tttgtgttag atccgtgttt 240
   gtgttagatc cgtgctgcta gcgttcgtac acggatgcga cctgtacgtc agacacgttc 300
   tgattgctaa cttgccagtg tttctctttg gggaatcctg ggatggctct agccgttccg 360
   cagacgggat cgatttcatg attttttttg tttcgttgca tagggtttgg tttgcccttt 420
   tcctttattt caatatatgc cgtgcacttg tttgtcgggt catcttttca tgcttttttt 480
   tgtcttggtt gtgatgatgt ggtctggttg ggcggtcgtt ctagatcgga gtagaattct 540
   gtttcaaact acctggtgga tttattaatt ttggatctgt atgtgtgtgc catacatatt 600
   catagttacg aattgaagat gatggatgga aatatcgatc taggataggt atacatgttg 660
   atgcgggttt tactgatgca tatacagaga tgcttttgtt cgcttggttg tgatgatgtg 720
   gtgtggttgg gcggtcgttc attcgttcta gatcggagta gaatactgtt tcaaactacc 780
   tggtgtattt attaattttg gaactgtatg tgtgtgtcat acatcttcat agttacgagt 840
   ttaagatgga tggaaatatc gatctaggat aggtatacat gttgatgtgg gttttactga 900
   tgcatataca tgatggcata tgcagcatct attcatatgc tctaaccttg agtacctatc 960
   tattataata aacaagtatg ttttataatt attttgatct tgatatactt ggatgatggc 1020
   atatgcagca gctatatgtg gattttttta gccctgcctt catacgctat ttatttgctt 1080
   ggtactgttt cttttgtcga tgctcaccct gttgtttggt gttacttctg caggtcgagt 1140
   ggccaccatg g                                                      1151

## Beschreibung

Die vorliegende Erfindung betrifft einen Promotor und dessen Verwendung sowie transgene Pflanzen.

Höhere Pflanzen enthalten ein Röhrensystem, das Xylem, in dem der aufsteigende Wasser- und Nährsalztransport stattfindet.

Die Beladung des Xylems in der Wurzel wird über das Xylemparenchym, welches die Xylemstränge umhüllt, reguliert. Die Xylemparenchymzellen der Wurzel stellen daher einen wichtigen Kontrollpunkt für den Stoffeintritt in das Xylem dar. Xylemparenchymzellen enthalten für diesen Zweck spezifische Protonenpumpen, Wasserkanäle und Ionenkanäle.

Mit seiner physiologischen Bedeutung für den Stoffwechsel eröffnet das Xylemparenchym eine Reihe von Möglichkeiten, um genetische Veränderungen an Pflanzen vorzunehmen. Einflüsse auf den Nährstofftransport könnten beispielsweise über bestimmte Proteine erfolgen, die hauptsächlich im Xylemparenchym aktiv sind.

Es sind bereits Promotoren bekannt, die u.a. in Xylemparenchymzellen aktiv sind. Allerdings ist die Aktivität dieser Promotoren entweder nicht ausschließlich auf Xylemparanchymzellen beschränkt (EP 1 207 204 A1), oder die Promotoren sind nicht hauptsächlich in den Wurzeln aktiv. So ist die Aktivität des Sultr2.1-Promoters auch im Blattphloem (Takahashi et al., 2000) nachweisbar, und der SOS1-Promotor ist auch in den Abschlußzellen der Wurzelspitzen aktiv (Shi et al., 2002). Der I2-Promotor des Resistenzgens I2 der Tomate ist hauptsächlich in den Xylemparenchymzellen des Sprosses und daneben auch in der Wurzel, dem Blatt und den Tomatenfrüchten aktiv (Mes et al., 2000). Der hochaffine Aminosäuretransporter AAP6 aus *A*. *thaliana* wird in den Xylemparenchymzellen aller Wurzel- und Blattgefäße durch den AAP6-Promotor exprimiert (Okumoto et al., 2002). RNA-Blot Analysen zeigen, daß das AAP6-Gen stark in Wurzeln, Sink- und Cauline-Blättern und schwächer im Sproß und den Blüten exprimiert wird (Rentsch et al., 1996).

Sakuta und Satoh, 2000, beschreiben glycinreiche CRGRP-Proteine, deren physiologische Funktion noch unklar ist, wobei angenommen wird, dass CRGRP-Proteine an der Wandverstärkung bei Xylemgefäßen beteiligt sind. Kloos et al., 2002, beschreiben u.a. die Isolierung und molekulare Analyse des Gens eines "Thaumatin-like"-Proteins (Tlp) aus der Zuckerrübe, das spezifisch in der Pfahlwurzel exprimiert wird. Promotoren sind aus beiden Druckschriften nicht bekannt. Aufgabe der vorliegenden Erfindung ist es daher, Stoffwechselleistungen einer Pflanze gezielt im Bereich des Xylemparenchyms von Wurzeln zu beeinflussen.

Erfindungsgemäß erfolgt die Lösung der gestellten Aufgabe durch einen Promotor gemäß den Merkmalen des Anspruchs 1.

Einige der in dieser Anmeldung verwendeten Begriffe werden nachfolgend näher erläutert:
Unter einem Promotor wird eine Nukleinsäuresequenz verstanden, die die Expression eines Gens gegebenenfalls in Abhängigkeit von endogenen und exogenen Faktoren steuert. Zu diesen Faktoren gehören z. B. Induktoren, Repressoren und ähnliche DNAbindende Proteine, aber auch Umwelteinflüsse. Ein Promotor kann aus mehreren Elementen bestehen. Er umfaßt jedoch mindestens ein regulatorisches Element, das für die Transkription des unter seiner Kontrolle stehenden Gens zuständig ist.

Ein Promotor, der in Xylemparenchymzellen von Pflanzenwurzeln aktiver ist als in anderen Zellen der Pflanze zeigt beispielsweise in Wurzeln eine durch RNA-Blots meßbare Aktivität, die bei vergleichbaren Versuchsbedingungen in oberirdischen Organen von Pflanzen wie Petiolen, Blättern und Blüten zu weniger als 20%, vorzugsweise zu weniger als 10% und insbesondere zu weniger als 5% nachweisbar ist. Diese Spezifität beschränkt sich licht auf einen bestimmten Untersuchungszeitpunkt, sondern ist grundsätzlich während der gesamten Vegetationszeit gegeben.

"Derivate" eines Promotors sind verkürzte oder verlängerte oder abschnittsweise identische Versionen oder Homologe dieses Promotors mit den gleichen oder im wesentlichen gleichen Eigenschaften.

"Pathogeninduzierbarkeit" bedeutet das Einwirken von äußeren Faktoren auf eine Pflanze, die eine Abwehrreaktion derselben nach sich zieht. Dabei kann es sich um Angriffe durch Insekten (Fraß), Bakterien, Pilze, Nematoden oder andere Pathogene handeln, aber auch um abiotische Einwirkungen, wie mechanische Verwundungen oder um Wasser- oder Salzstreß.

"Direkte antifungale Wirkung" bedeutet, daß Genprodukte unmittelbar antifungal wirken, indem sie z. B. Zellwände auflösen oder für Phytoalexinsynthasen codieren bzw. für Metabolite, die hemmend in den pilzlichen Stoffwechsel eingreifen.

"Indirekte antifungale Wirkung" bedeutet, daß Genprodukte die pflanzliche Genabwehr aktivieren. Zu diesen Genen gehören z. B. Resistenzgene, Komponenten der Signaltransduktion (wie Kinasen, Phosphatasen), Transkriptionsfaktoren oder Enzyme, die Signalsubstanzen produzieren (wie Ethylen bildende, Salicylsäure bildende oder Jasmonat bildende Enzyme, reaktive Sauerstoffspezies bildende Enzyme, Stickstoffmonoxyd bildende Enzyme).

Unter "Infektion" ist der früheste Zeitpunkt zu verstehen, bei dem der Stoffwechsel des Pilzes (bzw. das Wachstum des Pilzes) auf eine Penetration des Wirtsgewebes vorbereitet wird. Dazu gehören z. B. das Auswachsen von Hyphen oder die Bildung von spezifschen Infektionsstrukturen wie Penetrationshyphen und Appressorien.

Der Ausdruck "Homologie" bedeutet hierbei eine Homologie von mindestens 70 % auf DNA-Ebene, die gemäß bekannter Verfahren, z.B. der computergestützten. Sequenzvergleiche (S.F. Altschul et al., 1990), Basic Local Alignment search tool, J.Mol. Biol. 215: 403-410) bestimmt werden kann.

"Komplementäre Nukleotidsequenz" bedeutet bezogen auf eine doppelsträngige DNA, daß der zum ersten DNA Strang komplementäre zweite DNA Strang entsprechend den Basenpaarungsregeln die Nukleotidbasen aufweist, die zu den Basen des ersten Stranges korrespondieren.

Der hier verwendete Begriff "hybridisieren" bedeutet hybridisieren unter üblichen Bedingungen, wie sie in Sambrook et al. (1989) beschrieben sind, bevorzugt unter stringenten Bedingungen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Wenig stringente Hybridisierungsbedingungen sind beispielsweise: Hybridisieren in 4 x SSC bei 37 °C und anschließendes mehrfaches Waschen in 1 x SSC bei Raumtemperatur. "Stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C.

Die Erfindung wird nachfolgend und mit Bezug auf die Figuren und Beispiele näher erläutert.

Der erfindungsgemäße Promotor ist in Xylemparenchymzellen von Pflanzenwurzeln aktiv. Keine oder eine nur geringe Aktivität ist hingegen in oberirdischen Organen der betreffenden Pflanze nachweisbar. Diese Eigenschaft kann zur Verbesserung der Belade- und Entladeprozesse des Xylems der Wurzel und damit zur Verbesserung des Stoffwechsels genutzt werden. Unter Verwendung des Promotors lassen sich daher auch transgene Pflanzen und Teile dieser Pflanzen wie Samen herstellen.

Der erfindungsgemäße Promotor kann genutzt werden, um den Stickstoffstoffwechsel der Pflanzen zu verbessern. Dazu werden Transportproteingene für Nitrat und Aminosäuren in den Wurzelxylemparenchymzellen überexprimiert und die Beladung des Xylems mit den N-Verbindungen verstärkt. Eine weitere Verbesserung des N-Stoffwechsels stellt die reduzierte Einlagerung von "schädlichem Stickstoff" in die Speicherorgane der Pflanze dar. Erhöhte Konzentrationen an N-Verbindungen in Speicherorganen reduzieren oft den ernährungsphysiologischen Wert von Emteprodukten oder erschweren die Isolierung von Speicherstoffen wie Sucrose aus Zuckerrübenwurzeln. Eine reduzierte Einlagerung von "schädlichem Stickstoff" in die Wurzel kann durch eine verstärkte Abgabe von Aminosäuren an das Xylem und den Transport in die oberirdischen Pflanzenorgane erreicht werden.

Der erfindungsgemäße Promotor kann genutzt werden, um die Wasserstreßtoleranz der Pflanzen zu verbessern. Die Konzentration des Phytohormons Abscisinsäure (ABA) steigt in den Wurzeln in Reaktion auf das Trocknen der Erde an. ABA wird von der Wurzel über das Xylem in die Blätter transportiert, wo es ein Schließen der Stomata bewirkt. Durch eine Kontrolle der Expression des ABA-Transporters in den Xylemparenchymzellen kann der ABA-Eintritt in das Xylem reguliert und die Trockenstreßtoleranz der Pflanzen verbessert werden.

Der erfindungsgemäße Promotor kann genutzt werden, um die Konzentration der Kationen Natrium (Na⁺) und Kalium (K⁺) in der Wurzel von Zuckerrüben zu reduzieren. Die Na⁺/ K⁺-Konzentration bestimmt die Verarbeitungsqualität von Zuckerrüben im Hinblick auf die technische Zuckergewinnung (Schiweck et al., 1984). Den Na⁺- und K⁺-Transportmolektilen in den Xylemparenchymzellen der Wurzel kommt eine Schlüsselstellung für eine niedrige Na⁺/ K⁺-Konzentration in der Zuckerrübenwurzel zu. Mit Hilfe des Promotors können geeignete Verfahren durchgeführt werden, um die Na⁺/K⁺-Konzentration gering zu halten, beispielsweise die Überexpression des Na⁺/H⁺-Antiporters SOS1 (Shi et al., 2002) in den Xylemparenchymzellen. Andererseits ist die Beladung des Xylems mit K⁺ getrennt reguliert von der K⁺-Aufnahme aus dem Erdboden. Während AKT1 für die K⁺-Aufnahme aus dem Medium zuständig ist, erfolgt die K⁺-Beladung des Xylems durch das Transportmolekül SKOR (stelar K⁺ outward rectifier). Die gezielte Überexpression von SKOR (Gaymard et al. 1998) würde zu einem verstärkten Abtransport von K⁺ aus der Wurzel führen.

Der erfindungsgemäße Promotor kann auch genutzt werden, um die Krankheitsresistenz der Pflanzen zu verbessern. Zahlreiche bodenbürtige Pilze der Spezies *Fusarium oxysporum* oder *Verticillium* spp. nutzen das Xylem zur Ausbreitung in der Pflanze. Durch Kombination des pathogeninduzierbaren Promotors mit einem Gen, dessen Genprodukt eine direkte oder indirekte antifungale Wirkung ausübt, kann die weitere Ausbreitung eines Pilzes in dem Xylem unterbunden und dadurch eine Pilzresistenz verwirklicht werden. Dabei kommt der Pathogeninduzierbarkeit des Promotors eine besondere Rolle zu, um in dem Xylemparenchym eine für die Wirksamkeit des antifungalen Wirkprinzips kritische Expressionshöhe zu erreichen.

Virale Infektionen der Zuckerrübe sind oft nur auf ein Organ wie die Wurzel oder die oberirdischen Pflanzenteile beschränkt. So infiziert und koloniert das Virus BNYW in erster Linie die Rübenwurzel, und die Vergilbungsviren BMYV und BYV werden nur in Blättern gefunden. Der wurzelspezifische Promotor kann nun genutzt werden, um die auf dem gene silencing bzw. der antisense Technik beruhenden Virusresistenzkonzepte organspezifisch umzusetzen.

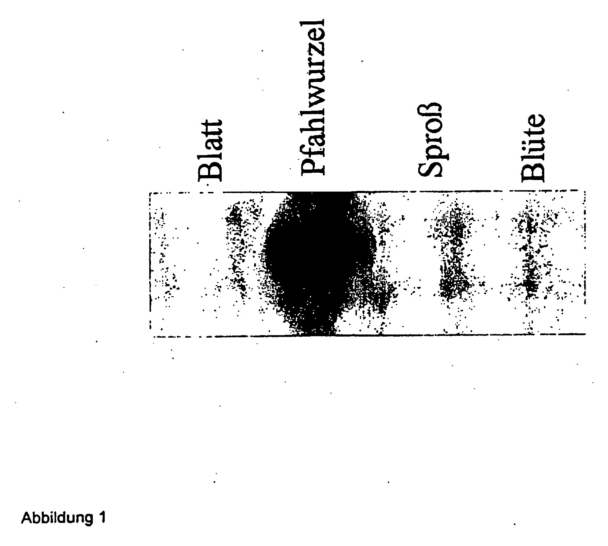

Abbildung 1 zeigt die wurzelspezifische Expression des Gens 2-1-88 durch ein RNA-Blot Experiment. Jeweils 10 µg Gesamtzell-RNA, die aus den Organen von 2-jährigen, blühenden Zuckerrüben isoliert worden waren, wurden in einem denaturierenden Formaldehyd-Agarosegel aufgetrennt. RNA wurde aus den Blättern, der Pfahlwurzel, dem Sproß und den Blüten isoliert. Das Smart cDNA Fragment 2-1-88 wurde als Hybridisierungssonde verwendet.

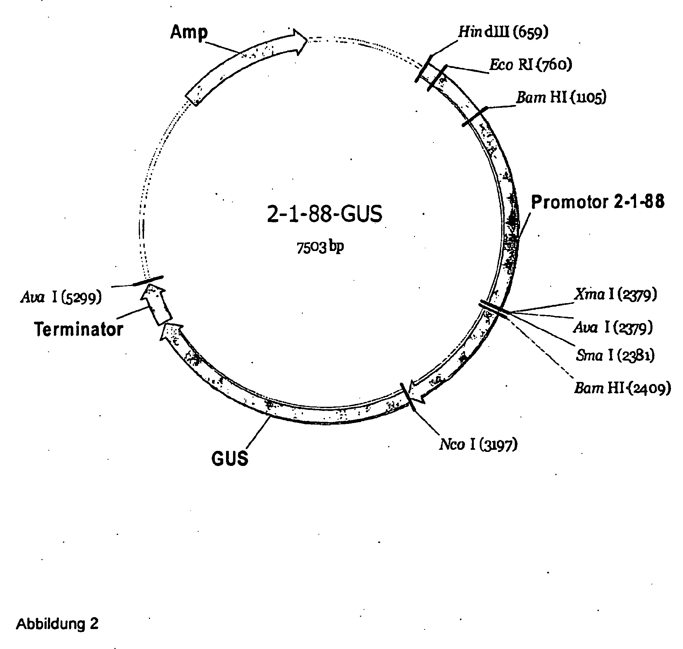

Abbildung 2 zeigt den Reportergenvektor 2-1-88-GUS mit einer translationellen Fusion zwischen dem Promotor (SEQ ID NO: 1) und dem GUS-Gen aus *E.coli*. Der Promotor in dem Vektor 2-1-88-GUS wurde als HindIII-NcoI Fragment inseriert und umfaßt die Nukleotidpositionen 1-2502 der Nukleotidsequenz SEQ ID NO: 1.

Abbildung 3 zeigt den Reportergenvektor 2-1-88-LUC mit einer translationellen Fusion zwischen dem Promotor (SEQ ID NO: 1) und dem Luciferase-Gen aus *Photinus pyralis.* Der Promotor 2-1-88 in dem Vektor 2-1-88-LUC umfaßt die Nukleotidpositionen 1-2502 der Nukleotidsequenz SEQ ID NO: 1.

Abbildung 4 zeigt die modulare Organisation des Promotors (SEQ ID NO: 1) und die Verteilung von cis-Elementen.

Abbildung 5 zeigt den binären Vektor 2-1-88-luc-kan, der für die Zuckerrübentransformation verwendet wurde.

Abbildung 6 zeigt die spezifische Luciferaseaktivität des Reportergenkonstruktes 2-1-88-luc-kan im Extrakt von Wurzeln und Blättern junger und alter transgener Zuckerrüben (WP4) sowie die Aktivität der nicht-transgenen Ausgangslinie (control). Die Skalierung der Y-Achse ist logarithmisch.

Abbildung 7 zeigt die räumliche Verteilung der Luciferaseaktivität im Wurzelquerschnitt der transgenen Zuckerrübenlinie WP4-15 im Vergleich zur nichttransgenen Ausgangslinie (control). Die Wurzelscheiben wurden beleuchtet fotografiert (links), und dann wurde die Lichtemission aus den Wurzelscheiben in der Dunkelheit dokumentiert (rechts). Die Lage und Stärke der Lichtemission korreliert mit der Aktivität des Promotors (SEQ ID NO: 1) in der Wurzel der Transformante WP4-15 (rechts unten). Die nichttransgene Ausgangslinie zeigt keine Lichtemission. (rechts oben).

Abbildung 8 zeigt eine mikroskopische Detailaufnahme der transgenen Wurzelscheibe WP4-15 aus Abbildung 7. In der linken Bildhälfte ist beleuchtete Wurzeloberfläche zu sehen. Die aus Phloem und Xylem aufgebauten Gefäßstränge erscheinen als schwarze Schlitze. In der rechten Bildhälfte ist die Lokalisation der Lichtemission um die Xylemstränge nachgewiesen. Diese Aufnahme wurde in der Dunkelheit aufgenommen, so daß nur die Lichtemission aus dem Objekt gemessen werden konnte.

Abbildung 9 zeigt eine Überlagerung der linken und der rechten Bildhälften aus Abbildung 8. Die Luciferase Reportergenaktivität erscheint in dieser Darstellung rot. Die Reportergenaktivität ist mit der Zellschicht zwischen Xylem und Phloem bzw. dem unmittelbaren Bereich um das Xylem auf die Xylemparenchymzellen beschränkt.

Abbildung 10 zeigt den Reportergenvektor pUBI-minimal mit dem Minimalpromotor des Ubiquitinpromotors aus Mais.

### Charakterisierung des Promotos 2-1-88

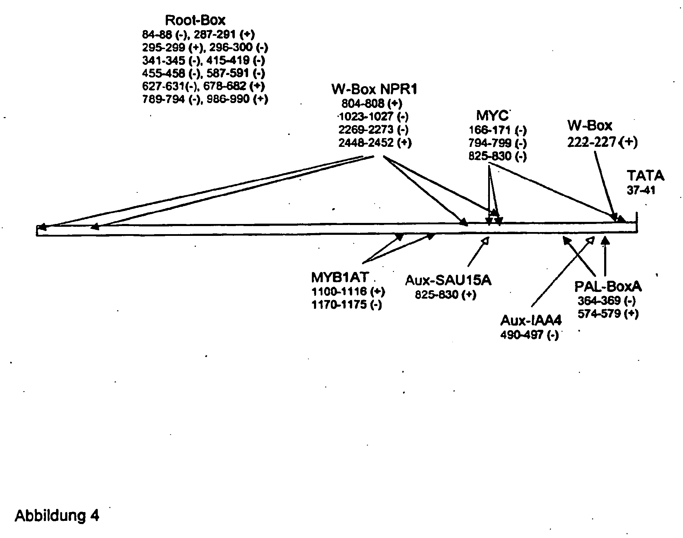

Der Promotor (SEQ ID NO: 1) stammt von einem bisher nicht bekannten Zuckerrübengen 2-1-88 und wird nachfolgend auch als 2-1-88-Promotor bezeichnet. Die Analyse dieses Promotors mit Hilfe der PLACE Datenbank (Higo et al., 1999) zeigt, daß zahlreiche cis-Elemente für Pathogeninduzierbarkeit, Wurzelspezifität sowie Wasser- und Kältestreßresponsivität nachweisbar sind (Tabelle 1). Ein charakteristisches, pathogenresponsives Element ist die W-Box, die einmal als "klassische" W-Box (Rushton et al., 2002) und viermal als W-Box NPR1 (Yu et al., 2001) in dem Promotor vorliegt (Abb. 4). Neben dem Kemmotif TTGACC bzw. TTGAC kommt jeweils den 15 Nukleotiden stromaufwärts und stromabwärts des W-Kernmotivs eine wichtige Bedeutung für die Pathogeninduzierbarkeit in Kombination mit dem Kemmotiv zu. Darüber hinaus ist die Box-A aus dem pathogen-induzierbaren PcPAL Promotor vorhanden. Die Wurzelspezifität des Promotors wird durch die zahlreichen wurzelspezifischen Motive (Wurzel Box ATATT) und die cis-Elemente für Auxinresponsivität (Aux-IAA4 und Aux-SAU15A) bestimmt (Guilfoyle et al., 1998). Die Wasser-, Salz- und Kältestreßinduzierbarkeit des Promotors ist auf die Anwesenheit der MYC und MYB Bindungsstellen (Abe et al., 2003), die in der ABA Signaltransduktion involviert sind, zurückzuführen.

### Fusion des 2-1-88-Promotors mit dem Luciferasegen aus Photinus pyralis

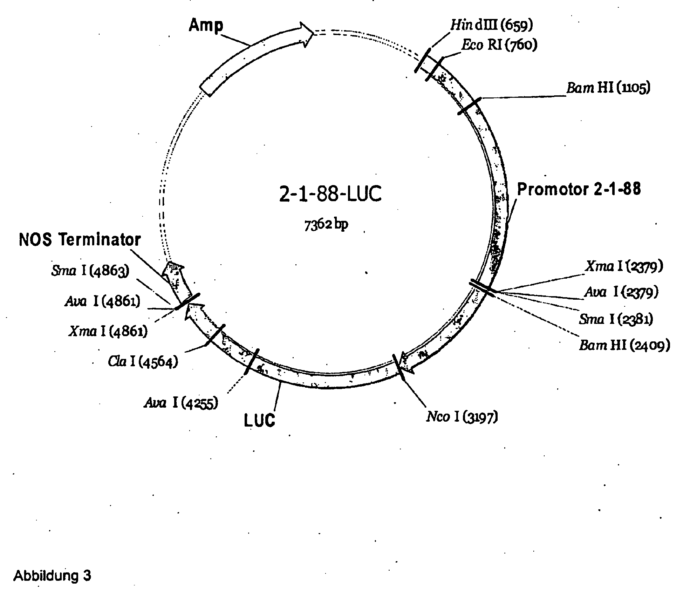

Um die Aktivität des 2-1-88-Promotors in Zuckerrüben nachzuweisen, wurde der Promotor mit dem Luciferasegen aus *Photinus pyralis* translationell fusioniert und in Zuckerrüben transformiert. Dazu wurde der Vektor 2-1-88-GUS zunächst durch eine SacI Spaltung mit anschließende T4-DNA-Polymerase-Auffüllreaktion linearisiert. Durch anschließenden Verdau mit NcoI wurde das GUS-Gen freigesetzt. In den so vorbereiteten Vektor wurde das Luciferasegen von *Photinus pyralis* (Promega, Mannheim Deutschland) als NcoI-BgIII (aufgefüllt) Fragment kloniert. Der resultierende Vektor trägt die Bezeichnung 2-1-88-LUC (Abb. 3) und enthält eine translationelle Fusion zwischen dem 2-1-88-Promotor und dem Luciferasegen.

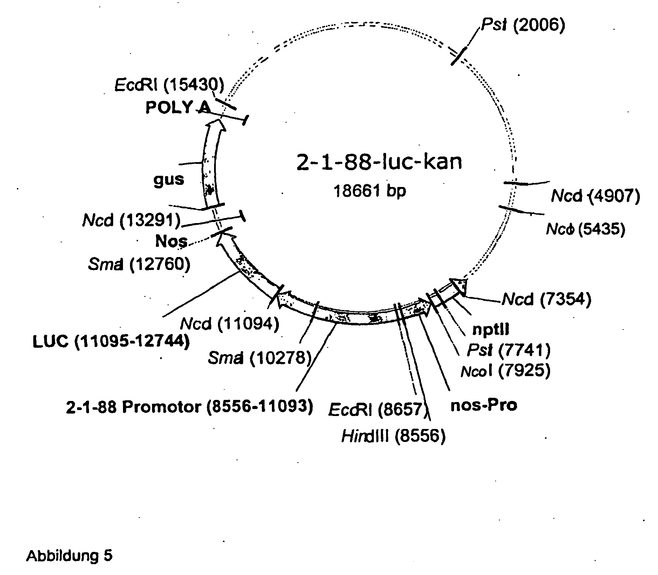

Für die Transformation der Zuckerrüben wurde die Expressionskassette bestehend aus dem 2-1-88-Promotor und dem Luciferasegen in den binären Vektor pGPTV-kan (Becker et al., 1992) umkloniert. Dazu wurde die 2-1-88-Promotor-Luciferasegen-Kombination mit Pvull und HindIII aus dem Vektor 2-1-88-LUC ausgeschnitten und in den mit HindIII und SmaI linearisierten binären Vektor pGPTV-kan kloniert. Der entstehende Vektor trägt die Bezeichnung 2-1-88-luc-kan (Abb. 5). Der Vektor 2-1-88-luc-kan wurde in den *Agrobacterium tumefaciens* Stamm C58C1 mit dem residenten Plasmid pGV2260 durch ein direktes DNA-Transformationsverfahren (An, 1987) transformiert. Die Selektion rekombinater *A*. *tumefaciens-*Klone erfolgte unter Verwendung des Antibiotikums Kanamycin (50 mg/l).

Die Transformation der Zuckerrüben erfolgte nach Lindsey et al. (1991) unter Verwendung des Antibiotikums Kanamycin. Die Transgenität der Pflanzen wurde durch PCR überprüft. Die Verwendung der Primer GTGGAGAGGCTATTCGGTA und CCACCATGATATTCGGCAAG führte zu der Amplifikation eines 553 bp großen DNA-Fragments aus dem *npt*II Gen. Die PCR wurde unter Verwendung von 10 ng genomischer DNA, einer Primerkonzentration von 0,2 µM bei einer Annealingtemperatur von 55°C in einem Multicycler PTC-200 (MJ Research, Watertown, USA) durchgeführt.

### Nachweis der 2-1-88-Promotoraktivität in Wurzeln transgener Zuckerrüben

Transgene Zuckerrüben, die mit dem Reportergenkonstrukt 2-1-88-luc-kan transformiert worden waren, wurden unter Gewächshausbedingungen angezogen. Die Aktivität des Promotors wurde in Wurzeln und Blättern von jungen und alten Zuckerrüben durch Reportergenmessungen analysiert.

Die *Photinus pyralis*-Luciferaseaktivität wurde mit dem Luciferase Assay System (Promega, Mannheim, Deutschland) in einem Sirius Luminometer (Berthold Detection System GmbH, Pforzheim, Deutschland) entsprechend den Herstellerangaben bestimmt. Für die Gewinnung eines für die Messung geeigneten Enyzmextraktes wurde zunächst das Gewicht der Gewebeproben ermittelt. Die Blattproben wurden unter Zugabe von Seesand mit dem 10-fachen Volumen (v/w) an Passive Lysis Buffer (PLB) in einem Mörser und die Wurzelproben in einem handelsüblichen Küchengerät (Warring Blender) homogenisiert. Der flüssige Überstand wurde in ein 1.5-ml-Eppendorfgefäß überführt und 5 min bei 4 °C und 20 000 g abzentrifugiert. Der klare Überstand wurde abgenommen und jeweils 10 µl Rohextrakt für die *Photinus*-Luciferaseaktivitätsmessung eingesetzt.

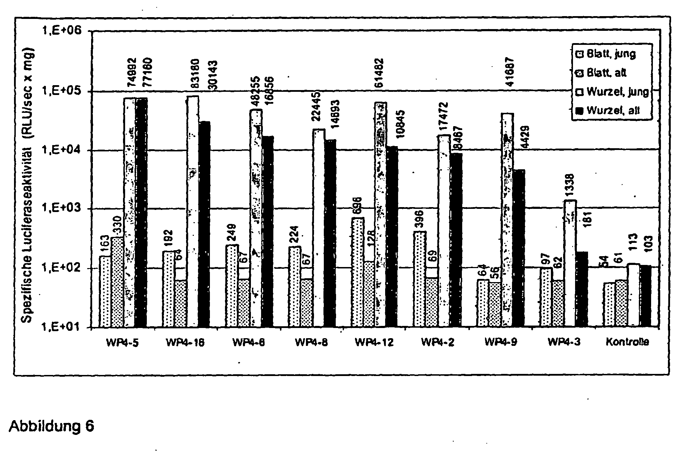

Während der Promotor (SEQ ID NO: 1) in den jungen und alten Wurzeln von 9 unabhängigen Transformanten stark bzw. sehr stark exprimiert wird, war die Reportergenaktivität bei 7 Transformanten (WP4-2, WP4-5, WP4-6, WP4-8, WP4-9, WP4-12, WP4-16) im Vergleich zur nichttransgenen Ausgangslinie in den Blättern junger Pflanzen kaum und in alten Pflanzen nicht nachweisbar (Abbildung 6). Der Promotor (SEQ ID NO: 1) wird nach den Ergebnissen der Reportergenstudie daher in Übereinstimmung mit den RNA-Blot-Studien hauptsächlich in der Wurzel der Zuckerrübe exprimiert.

### Der 2-1-88-Promotor ist in den Xylemparenchymzellen der Zuckerrübenwurzel spezifisch aktiv

Um die räumliche Verteilung der Promotoraktivität in den Zuckerrübenwurzeln zu analysieren, wurden Quer- und Längsschnitte von Rübenwurzeln angefertigt und die Wurzelschnitte 2-4 h in einer Lösung von 100 µM Luciferin + 5% DMSO bei Raumtemperatur inkubiert. Anschließend wurde die Lichtemission aus den Wurzelschnitten, die auf die Luciferaseaktivität zurückzuführen ist, mit Hilfe einer MicroMAX Digital CCD Kamera (Visitron Systems GmbH, Puchheim, Deutschland) nachgewiesen.

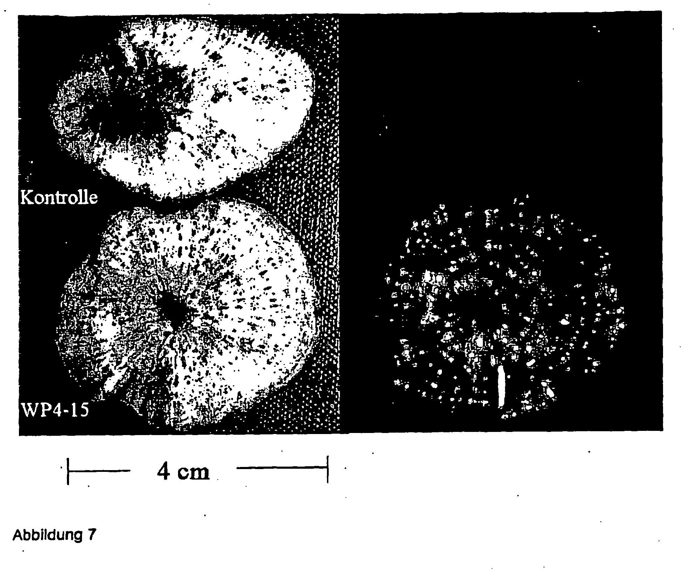

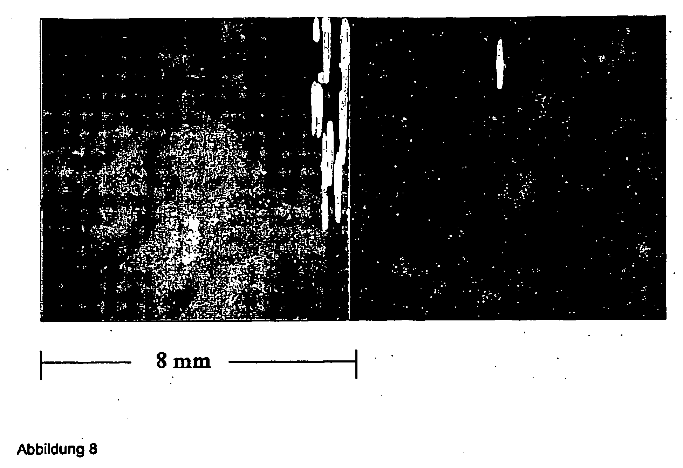

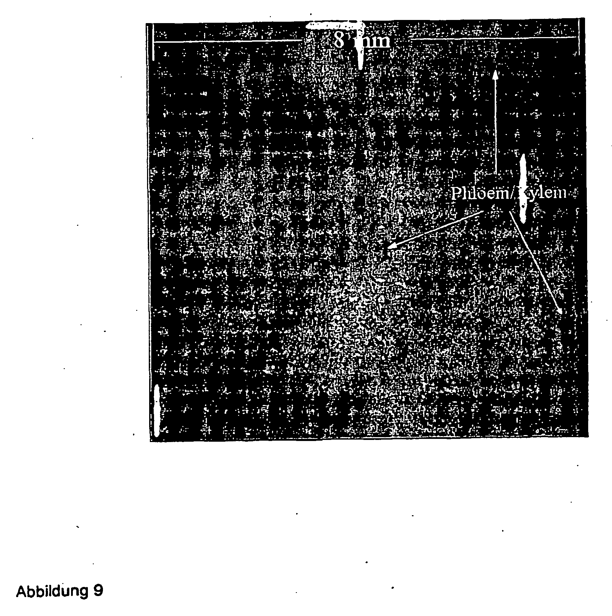

Die Analyse der Querschnitte zeigt, daß die Promotoraktivität mit der Lage der Gefäße in den einzelnen Kambiumringen der Wurzel assoziiert ist (Abb. 7). Für eine detailiertere Analyse wurde das Stereomikroskop Stemi 2000 (Zeiss, Deutschland) mit Hilfe eines HRD Mikroskop Adaptors in die Kamera eingebaut. Nach der mikroskopischen Analyse ist die Promotoraktivität auf die Xylemparenchymzellen zwischen Xylem und Phloem bzw. die das Xylem umgebenden Xylemparenchymzellen beschränkt (Abb. 8 und 9).

Mit dem erfindungsgemäßen Promotor lassen sich transgene Pflanzen mit besonderen Eigenschaften herstellen:
a. Verbesserte Belade- und Entladeprozesse des Xylems in der Wurzel
b. Verbesserte Stickstoffversorgung
c. Verringerte Akkumulation von "schädlichem Stickstoff" in der Wurzel
d. Verbesserte Salzresistenz
e. Verbesserte Trockenstreßresistenz
f. Verbesserte Frosttoleranz
g. Veränderte Na+/K+-Konzentration in der Wurzel
h. Erhöhte Resistenz/Toleranz gegenüber Pathogenen

### Der 2-1-88-Promotor wird in den Xylemparenchvmzellen durch biotischen und abiotischen Streß induziert

Transgene WP4 Zuckerrüben, die mit dem Reportergenkonstrukt 2-1-88-luc-kan transformiert worden waren, wurden im Gewächshaus mit dem parasitäten Pilz *Fusarium oxysporum* f. sp. betae infiziert. *Fusarium oxysporum* f. sp. *betae* ist ein. Gefäßpathogen der Rübe, der sich im Xylemsystem der Pflanze ausbreitet und die Fusarium-Welke hervorruft. 4 Wochen nach der Infektion wurde die Luciferase Reportergenaktivität in den infizierten Wurzeln quantifiziert. Die Reportergenaktivität war in den infizierten Wurzeln im Vergleich zu den nichtinfizierten transgenen Pflanzen deutlich erhöht. Der 2-1-88-Promotor wird somit durch Pathogenbefall induziert. Die Analyse der räumlichen Verteilung der Reportergenaktivität mit Hilfe der CCD-Kamera zeigte, daß die Promotoraktivität in den Xylemparenchamzellen, die die infizierten Xylembündel umgeben, deutlich erhöht war.

### Veränderung des Stickstoffmetabolismus von Pflanzen

Der Stickstoffmetabolismus von Pflanzen kann durch die Verwendung des erfindungsgemäßen Promotors in vielfältiger Hinsicht verbessert werden. Die spezifische Erhöhung oder Verringerung der Zahl geeigneter Transportproteine in den Xylemparenchymzellen der Wurzel verbessert die Aufnahme und den Transport von N-Verbindungen in der Pflanze.

Durch die wurzelspezifische Expression von Transportproteingenen für Nitrat-Ionen im Xylemparenchym kann die Stickstoffaufnahme aus dem Boden gesteigert und die Nutzung von N-Dünger verbessert werden. Der verbesserte Nitrattransport in die oberirdischen Pflanzenteile führt zu einer verstärkten Aminosäureproduktion in den Blättern. Einer effizienteren Nutzung der in der Wurzel bereits zu Aminosäuren reduzierten N-Verbindungen dient die xylemparenchymspezifische Expression von Aminosäuretransportem durch den Promotor.

Eine weitere Verbesserung des N-Stoffwechsels stellt die reduzierte Einlagerung von "schädlichem Stickstoff" in die Speicherorgane der Pflanze dar. Erhöhte Konzentrationen an N-Verbindungen in Speicherorganen reduzieren oft den ernährungsphysiologischen Wert von Ernteprodukten oder erschweren die Isolierung von Speicherstoffen wie Sucrose aus Zuckerrübenwurzeln. Eine reduzierte Einlagerung von "schädlichem Stickstoff" in die Zuckerrübenwurzel kann durch eine verstärkte Abgabe von Aminosäuren aus der Wurzel in die oberirdischen Pflanzenteile Ober das Xylem erreicht werden. Dazu werden entsprechende Aminosäuretransporter überexprimiert.

### Erhöhung der Toleranz gegenüber phytopathogenen Viren

Zahlreiche phytopathogene Viren der Zuckerrübe zeigen eine Organspezifität, d.h. die virale Vermehrung erfolgt gewöhnlich nicht in der ganzen Pflanze, sondern nur in einem bestimmten Organ oder Gewebetyp. Ebenso sind die Schäden, die die virale Infektion hervorruft, in der Regel auf das befallene Organ beschränkt. Virale Krankheitserreger der Zuckerrübe mit einer Organspezifität sind z. B. BNYW mit der Präferenz für die Wurzel und BMYV und BYV mit der Beschränkung auf Rübenblätter.

Der erfindungsgemäße Promotor kann genutzt werden, um eine wurzelspeziflsche BNYW-Resistenz in der Zuckerrübe zu entwickeln. Dazu wird für die Umsetzung der gene-silencing-abhängigen Virusresistenzstrategie eine native oder mutagenisierte DNA-Teilsequenz des viralen BNYVV-Genoms mit dem Promotor kombiniert. Die Kombination zwischen der Promotorsequenz und der viralen DNA-Sequenz wird so gestaltet, daß die Transkription der BNYVV-Sequenz zu einem gegen das BNYVV wirksamen gene silencing führt. Die Wirksamkeit des Ansatzes wird über eine Bestimmung des Virustiters in den Pflanzen unter Nutzung eines ELISA-Tests, der gegen das Hüllprotein des BNYW gerichtet ist, bestimmt.

### Erhöhung der Toleranz transgener Pflanzen gegenüber bodenbürtigen phytopathogenen Pilzen

Der erfindungsgemäße Promotor kann auch genutzt werden, um in Kombination mit einem Gen oder einer Genkombination eine direkte oder indirekte, antifungale Wirkung in den Wurzeln von Pflanzen auszuprägen. Die antifungale Wirkung führt zu einer erhöhten Pilzresistenz oder Pilztoleranz speziell gegenüber Gefäßparasiten wie *Fusarium oxysporum* spp.

Der Promotor wird hierzu mit Genen der Pathogenabwehr, deren Genprodukte eine direkte oder indirekte antifungale Wirkung haben, translationell oder transkriptionell fusioniert. Die Promotorgenkombinationen werden in den binären Transformationsvektor pGPTV kloniert und durch *A. tumefaciens* vermittelte Transformation in Zuckerrübe, Kartoffel oder Raps transformiert. Die Transgenität der Pflanzen wird wie beschrieben durch PCR überprüft und die Expression der Gene in den Wurzeln durch RNA-Blot-Studien verifiziert. Die erhöhte Pilzresistenz der Pflanzen wird bei Pilzresistenzprüfungen beobachtet.

Überraschenderweise führt die wurzel- und xylemparenchymspezifische, induzierte Expression von Pathogenabwehrgenen nicht zu der bei einer konstitutiven Expression in der gesamten Pflanze oft beobachteten Kleinwüchsigkeit oder Ertragsminderung (Heil and Baldwin, 2002). Ein weiterer Vorteil der lediglich wurzelspezifischen Genexpression ist, daß die resistenz-vermittelnden Genprodukte nur in dem zu schützenden Organ gebildet werden. Für die Vermarktung bestimmte Pflanzenteile wie Rapssamen bleiben damit frei von sämtlichen Transgenprodukten.

Gene, deren Produkte eine indirekte, antifungale Wirkung haben, benötigen eine besonders sorgfältige Expressionskontrolle, um negative Folgen einer ungewollten Aktivierung der pflanzlichen Abwehr zu vermeiden. Ein Beispiel für eine indirekte, antifungale Wirkung ist der Effekt, der von der Coexpression eines pflanzlichen Resistenz-(R)-Gens in Kombination mit einem Avirulenz-(avr)-Gen oder der Überexpression eines autoaktivierten R-Gens in einer pflanzlichen Zelle ausgeht. Die gemeinsame Expression von R- und avr-Gen oder die singuläre Expression eines autoaktivierten R-Gens führt zu einer intensiven Aktivierung der pflanzlichen Abwehr und bedarf einer strikten Regulation. Die Regulation wird erreicht, indem entweder das R-Gen oder das avr-Gen unter die Kontrolle des erfindungsgemäßen Promotors gestellt wird.

### Nutzung des erfindungsgemäßen Promotors in Monokotyledonen

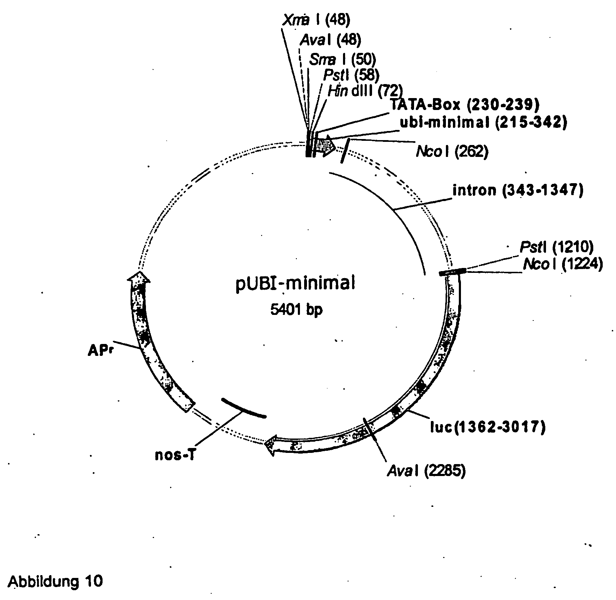

Obwohl der Promotor auch in Monokotyledonen aktiv ist, wird eine optimale Aktivität des Promotors in Monokotyledonen dann erreicht, wenn der Zuckerrüben-Minimalpromotor inklusive TATA-Box durch den Minimalpromotor des Ubiquitinpromotors aus Mais einschließlich dem Intron (SEQ ID NO: 2) ersetzt wird. Dazu wird die SEQ ID NO: 1 von Position 1-2459 durch PCR amplifiziert und in den mit Smal geschnittenen Vektor pUBI-minimal (Abb. 10) kloniert.

### Referenz:

Abe, H., Urao, T., Ito T, Seki, M., Shinozaki, K., and Yamaguchi-Shinozaki K. (2003). Arabidopsis AtMYC2 (bHLH) and AtMYB2 (MYB) function as transcriptional activators in abscisic acid signaling: Plant Cell. 15(1):63-78.
Altschul, S.F. et al. (1990). Basic Local Alignment search tool, J.Mol. Biol. 215: 403-410
An, G. (1987). Binary Ti vectors for plant transformation and promoter analysis. Methods Enzymol. 153, 292-305.
Becker D, Kemper E, Schell J, and Masterson R. (1992). New plant binary vectors with selectable markers located proximal to the left T-DNA border. Plant Mol Biol. 20(6):1195-7.
Elmayan, T. and Tepfer M. (1995). Evaluation in tobacco of the organ specificity and strength of the rolD promoter, domain A of the 35S promoter and the 35S2 promoter. Transgenic Res. 4(6):388-96.
Gaymard F, Pilot G, Lacombe B, Bouchez D, Bruneau D, Boucherez J, Michaux-Ferriere N, Thibaud JB, Sentenac H. (1998). Identification and disruption of a plant shaker-like outward channel involved in K+ release into the xylem sap. Cell 94, 647-655.
Guilfoyle, T., Hagen, G., Ulmasov, T., and Murfett, J. (1998). How does auxin turn on genes? Plant Physiol.118(2):341-7.
Heil, M. and Baldwin, I. T. (2002). Fitness costs of induced resistance: emerging experimental support for a slippery concept. Trends Plant Sci. 2002 Feb;7(2):61-7.
Higo, K., Ugawa, Y., Iwamoto, M. and Korenaga, T. (1999). Plant cis acting regulatory DNA elements (PLACE) database:1999. Nucleic Acid Research 27: 297-300. Kloos, D.U. et al. (2002), Isolation and molecular analysis of six taproot expressed genes from sugar beet, J. Exp. Bot. 53 (373): 1533-1534. '
Lindsey, K,. Gallois, P., and Eady, C. (1991) Regeneration and transformation of sugar beet by Agrobacterium tumefaciens. Plant Tissue Culture Manual B7: 1-13; Kluwer Academic Publishers.
Logemann, E., Pamiske. M., Hahlbrock, K. (1995). Modes of expression and common structural features of the complete phenylalanine ammonia-lyase gene family in parsley. Proc Natl Acad Sci USA. 1995 92(13):5905-9.
Mes JJ, van Doom AA, Wijbrandi J, Simons G, Comelissen BJ, and Haring MA. (2000). Expression of the Fusarium resistance gene I-2 colocalizes with the site of fungal containment. Plant J. 23(2):183-93.
Okumoto S, Schmidt R, Tegeder M, Fischer WN, Rentsch D, Frommer WB, and Koch W. (2002). High affinity amino acid transporters specifically expressed in xylem parenchyma and developing seeds of Arabidopsis. J Biol Chem. 277(47):45338-46.
Rentsch D., Himer, B, Schmelzer, E., and Frommer W.B. (1996). Salt stress-induced proline transporters and salt stress-repressed broad specificity amino acid permeases identified by suppression of a yeast amino acid permease-targeting mutant. Plant Cell. 1996 Aug;8(8):1437-46.
Rushton, P.J., Reinstadler. A., Lipka. V, Lippok, B., and Somssich, I, E. (2002). Synthetic plant promoters containing defined regulatory elements provide novel insights into pathogen- and wound-induced signaling. Plant Cell. 14(4):749-62.
Sambrook, J., Fritsch, E..F., and Maniatis, T (1989). In Molecular Cloning, A Laboratrory Manual (Cold Spring Harbor Laboratory Press, New York).
Sakuta C. und Satoh S. (2000), Vascular Tissue-Specific Gene Expression of Xylem Sap Glycine-Rich Proteins in Root and Their Localization in the Walls of Metaxylem Vessels in Cucumber, Plant Cell Physiol 41 (5): 627-638.
Schiweck, H., Kozianowski, G., Anderlei, J. and Burba, M. (1984). Errechnung der Dicksaft-Nichtzuckermasse aus Rübenanalysen. Zuckerindustrie 119, 268-282.
Shi H, Quintero FJ, Pardo JM, Zhu JK. (2002). The putative plasma membrane Na(+)/H(+) antiporter SOS1 controls long-distance Na(+) transport in plants. Plant Cell 14, 465-477.
Takahashi H, Watanabe-Takahashi A, Smith FW, Blake-Kalff M, Hawkesford MJ, and Saito K. (2000). The roles of three functional sulphate transporters involved in uptake and translocation of sulphate in Arabidopsis thaliana. Plant J.23(2):171-82.
Yu, D., Chen, C., and Chen, Z. (2001). Evidence for an important role of WRKY DNA binding proteins in the regulation of NPR1 gene expression. Plant Cell. 2001 (7):1527-40.

**Tabelle 1: Organisation der cis-Elemente in dem Promotor 2-1-88**

| | | |
|---|---|---|
| Die Positionsangaben beziehen sich auf den Transkriptionsstartpunkt (+1). | | |
| ¹(+)= direkte Orientierung zur TATA-Box, (-) = reverse Orientierung zur TATA Box. | | |

| Cis-Element (Sequenz) | Position¹ | Funktion |
|---|---|---|
| TATA Box (TATAAA) | 36-41 (+) | Bindungsstelle des basalen Transkriptionsfaktor-Komplexes |
| Wurzel-Box | 84-88 (-), 287-291 (+) | Element für Wurzelspezifität aus dem rol D |
| (ATATT) | 295-299 (+), 296-300 (-) | Promotor |
| | 341-345 (-), 415-419 (-) | (Elmayan and Tepfer, 1995). |
| | 455-458 (-), 587-591 (-) | |
| | 627-631(-), 678-682 (+) | |
| | 789-794 (-), 986-990 (+) | |
| MYC | 166-171 (-) | MYB Core-Bindungsstelle aus dem trockenstreßresponsiven Promotor rd22 und anderen Genen (CBF3), beteiligt an der ABA- und Kältesignaltransduktion (Abe et al., 2003) |
| Consensus | 794-799 (-) | |
| (CANNTG, | 825-830 (-) | |
| N=A,T,C,G) | | |
| W-Box (TTGACC) | 222-227 (+) | Core-Motif der WRKY Bindugsstelle von PcPR1-1 und PcPR1-2, ausreichend für die Pathogeninduktion von synthetischen Promotoren (Rhushton et al., 2002) |
| PAL-BoxA | 364-369 (-) | Box A aus dem pathogenresponsiven PcPAL |
| (CCGTCC) | 574-579 (+) | Promotor (Logemann et al., 1998) |
| Aux-IAA4 | 490-497 (-) | Auxin responsives cis-Element aus dem PS-IAAA4/5 |
| (G/TGTCCC AT) | | Promotor nach Guilfoyle et al. (1998) |
| W-Box NPR1 | 804-808 (+) | Core-Motif der WRKY Bindungsstelle von AtNPR1, verantwortlich für die Pathogeninduktion (Yu et al., 2001) |
| (TTGAC) | 1023-1027 (-) | |
| | 2269-2273 (-) | |
| | 2448-2452 (+) | |
| Aux-SAU15A | 825-830 (+) | Auxin responsives cis-Element aus dem SAUR15A |
| (CATATG) | | Promotor nach Guilfoyle et al. (1998) |
| MYB1AT | 1100-1116 (+) | MYB Bindungsstelle aus dem trockenstreßresponsiven Promotor rd22, beteiligt an der ABA-Signaltransduktion (Abe et al., 2003) |
| (A/TAACCA) | 1170-1175 (-) | |

## Patentansprüche

1. Promotor, umfassend eine Nukleotidsequenz gemäß SEQ ID NO:1 oder eine zu der Nukleotidsequenz gemäß SEQ ID NO: 1 komplementäre Nukleotidsequenz oder eine Nukleotidsequenz, die mit der Nukleotidsequenz gemäß SEQ ID NO: 1 oder einer zu der Nukleotidsequenz gemäß SEQ ID NO: 1 komplementären Nukleotidsequenz unter stringenten Bedingungen hybridisiert, wobei der Promoter eine gewebespezifische Aktivität aufweist und in Xylemparenchymzellen von Pflanzenwurzeln aktiver ist als in anderen Zellen der Pflanze.

2. Verkürzte oder verlängerte oder abschnittsweise identische Versionen oder Homologe mit mindestens 70% Homologie auf DNA-Ebene eines Promotors nach Anspruch 1, die eine gewebespezifische Aktivität aufweisen und in Xylemparenchymzellen von Pflanzenwurzeln aktiver sind als in anderen Zellen der Pflanze.

3. Vektor oder mobiles genetisches Element, enthaltend einen Promotor nach Anspruch 1 oder verkürzte oder verlängerte oder abschnittsweise identische Versionen oder Homologe mit mindestens 70% Homologie auf DNA-Ebene eines Promotors nach Anspruch 2.

4. Eukaryotische oder prokaryotische Wirtszelle, die mit einem Promotor nach Anspruch 1 oder verkürzten oder verlängerten oder abschnittsweise identischen Versionen oder Homologen mit mindestens 70% Homologie auf DNA-Ebene eines Promotors nach Anspruch 2 transformiert ist.

5. Transgene Pflanze oder deren Teile, die mit einem Promotor nach Anspruch 1 oder verkürzten oder verlängerten oder abschnittsweise identischen Versionen oder Homologen mit mindestens 70% Homologie auf DNA-Ebene eines Promotors nach Anspruch 2 transformiert sind.

6. Transgene Pflanze nach Anspruch 5. **dadurch gekennzeichnet, daß** die Pflanze *Beta vulgaris* ist.

7. Transgene Samen von Pflanzen nach Anspruch 5 oder 6 wobei die Pflanze mit einem Promotor nach Anspruch 1 oder verkürzten oder verlängerten oder abschnittsweise identischen Versionen oder Homologen mit mindestens 70% Homologie auf DNA-Ebene eines Promotors nach Anspruch 2 transformiert ist.

8. Verwendung eines Promotors nach Anspruch 1 oder von verkürzten oder verlängerten oder abschnittsweise identischen Versionen oder Homologen mit mindestens 70% Homologie auf DNA-Ebene eines Promotors nach Anspruch 2 zur Herstellung einer transgenen Pflanze mit einer oder mehreren der folgenden Eigenschaften:
a. Verbesserte Belade- und Entladeprozesse des Xylems in der Wurzel
b. Verbesserte Stickstoffversorgung
c. Verringerte Akkumulation von "schädlichem Stickstoff" in der Wurzel
d. Verbesserte Salzresistenz
e. Verbesserte Trockenstreßresistenz
f. Verbesserte Frosttoleranz
g. Veränderte Na+/K+-Konzentration in der Wurzel
h. Erhöhte Resistenz/Toleranz gegenüber Pathogenen

## Claims

1. Promoter comprising a nucleotide sequence according to SEQ ID NO: 1 or a nucleotide sequence complementary to the nucleotide sequence according to SEQ ID NO: 1 or a nucleotide sequence hybridizing under stringent conditions to the nucleotide sequence according to SEQ ID NO: 1 or to a nucleotide sequence complementary to the nucleotide sequence according to SEQ ID NO: 1, wherein the promoter exhibits a tissue specific activity and is more active in xylem parenchyma cells of plant roots than in other cells of the plant.

2. Shortened or elongated or sectionwise identical versions or homologs with at least 70% homology on DNA level of the promoter according to claim 1 which have a tissue specific activity and are more active in xylem parenchyma cells of plant roots than in other cells of the plant.

3. Vector or mobile genetic element, containing a promoter according to claim 1 or shortened or elongated or sectionswise identical versions or homologs with at least 70% homology on DNA level of a promoter according to claim 2.

4. Eukaryotic or prokaryotic host cell transformed with a promoter according to claim 1 or with shortened or elongated or sectionwise identical versions or homologs with at least 70% homology on DNA level of a promoter according to claim 2.

5. Transgenic plant or parts thereof, transformed with a promoter according to claim 1 or shortened or elongated or sectionwise identical versions or homologs with at least 70% homology on DNA level of a promoter according to claim 2.

6. Transgenic plant according to claim 5, thereby characterized that the plant is *Beta vulgaris*

7. Transgenic seeds of plants according to claim 5 or 6, wherein the plant is transformed with a promoter according to claim 1 or shortened or elongated or sectionwise identical versions or homologs with at least 70% homology on DNA level of a promoter according to claim 2.

8. Use of a promoter according to claim 1 or of shortened or elongated or sectionwise identical versions or homologs with at least 70% homology on DNA level of a promoter according to claim 2 for the production of a transgenic plant with one or more of the following characteristics:
a) Improved loading and unloading processes of the xylem in the root
b) Improved nitrogen supply
c) Reduced accumulation of "detrimental nitrogen" in the root
d) Improved salt resistance
e) Improved drought resistance
f) Improved frost tolerance
g) Modified Na⁺/K⁺ concentration in the root
h) Elevated resistance I tolerance to pathogens

## Revendications

1. Promoteur, comprenant une séquence de nucléotides selon la SEQ ID NO: 1 ou une séquence de nucléotides complémentaire à la séquence de nucléotides selon la SEQ ID NO:1 ou une séquence de nucléotides qui s'hybride avec la séquence de nucléotides selon la SEQ ID NO:1 ou avec une séquence de nucléotides complémentaire à la séquence de nucléotides selon la SEQ ID NO:1 dans des conditions rigoureuses, le promoteur ayant une activité spécifique au tissu et étant plus actif dans les cellules de xylème parenchyme des racines des plantes que dans les autres cellules de la plante.

2. Versions ou homologues raccourcis ou rallongés ou identiques par sections ayant une homologie d'au moins 70 % sur le plan de l'ADN d'un promoteur selon la revendication 1, qui présentent une activité spécifique au tissu et sont plus actifs dans les cellules de xylème parenchyme des racines des plantes que dans les autres cellules de la plante.

3. Vecteur ou élément génétique contenant un promoteur selon la revendication 1 ou versions ou homologues raccourcis ou rallongés ou identiques par sections ayant une homologie d'au moins 70 % sur le plan de l'ADN d'un promoteur selon la revendication 2.

4. Cellule hôte eucaryote ou procaryote qui est transformée avec un promoteur selon la revendication 1 ou des versions ou homologues raccourcis ou rallongés ou identiques par sections ayant une homologie d'au moins 70 % sur le plan de l'ADN d'un promoteur selon la revendication 2.

5. Plantes transgéniques ou parties de celles-ci qui sont transformées avec un promoteur selon la revendication 1 ou des versions ou homologues raccourcis ou rallongés ou identiques par sections ayant une homologie d'au moins 70 % sur le plan de l'ADN d'un promoteur selon la revendication 2.

6. Plantes transgéniques selon la revendication 5, **caractérisées en ce que** la plante est la *Beta vulgaris.*

7. Semences transgéniques de plantes selon la revendication 5 ou 6, la plante étant transformée avec un promoteur selon la revendication 1 ou des versions ou homologues raccourcis ou rallongés ou identiques par sections ayant une homologie d'au moins 70 % sur le plan de l'ADN d'un promoteur selon la revendication 2.

8. Utilisation d'un promoteur selon la revendication 1 ou de versions ou homologues raccourcis ou rallongés ou identiques par sections ayant une homologie d'au moins 70 % sur le plan de l'ADN d'un promoteur selon la revendication 2 pour la production de plantes transgéniques ayant une ou plusieurs des propriétés suivantes :
a) Meilleurs processus de chargement et déchargement du xylème dans la racine
b) Meilleure alimentation en azote
c) Réduction de l'accumulation de l'"azote nocif" dans la racine
d) Meilleure résistance au sel
e) Meilleure résistance au stress de sécheresse
f) Meilleure tolérance au gel
g) Modification de la concentration de Na⁺/K⁺ dans la racine
h) Résistance/tolérance aux pathogènes accrue
